# EUROPEAN PATENT APPLICATION

(11) **EP 1 867 330 A1**
(43) Date of publication of application: **19.12.2007**
(21) Application number: 06730570.6
(22) Date of filing: 30.03.2006
(51) Int. Cl.: A61K 31/375, A61K 9/14, A61K 9/20, A61K 9/34, A61K 9/48, A61K 31/7084, A61K 47/18, A61P 3/02

(54) **METHOD OF IMPROVING THE STORAGE STABILITY OF SUBSTANCE**

(30) Priority: 30.03.2005 JP 2005098210
(71) Applicant: KYOWA HAKKO KOGYO CO., LTD., Chiyoda-ku, Tokyo 100-8185 (JP)
(72) Inventor: KAMIYA, Toshikazu c/o Healthcare Products Development Center, Ibaraki 305-0841 (JP); KIMURA, Masao c/o Healthcare Products Development Center, Ibaraki 305-0841 (JP); SAKAI, Yasushi c/o Healthcare Products Development Center, Ibaraki 305-0841 (JP)
(74) Representative: Harding, Charles Thomas
(86) International application number: PCT/JP2006/306622
(87) International publication number: WO 2006/106805

(57) **Abstract**

The present invention relates to a method of improving storage stability of reduced form of nicotinamide adenine dinucleotide, reduced form of nicotinamide adenine dinucleotide phosphate, or a salt thereof, or ascorbic acid, an ascorbic acid derivative, or a salt thereof, which comprises allowing the substance to coexist with an L-arginine acidic amino acid salt, a composition containing the substance and an L-arginine acidic amino acid salt, a process for producing the composition, and a method of storing the substance in the presence of an L-arginine acidic amino acid salt.

## Description

### Technical Field

The present invention relates to a method for improving storage stability of reduced form of nicotinamide adenine dinucleotide (abbreviated to "NADH" hereafter), reduced form of nicotinamide adenine dinucleotide phosphate (abbreviated to "NADPH" hereafter) [hereinafter, NADH and NADPH are together referred to as "NAD(P)H"], or a salt thereof, or ascorbic acid, an ascorbic acid derivative, or a salt thereof, a composition containing such a substance, a process for producing the composition, and a method of storing the substance.

### Background Art

NAD(P)H and ascorbic acid are useful substances as raw materials for health foods and medicines but are unstable at room temperature and thus have the problem that the residual ratios during storage are lowered, i.e., the problem of low storage stability.

Therefore, in producing products containing such substances, consideration is given to improvement in the storage stability of the substances.

For example, a method known as a method of improving storage stability of NAD(P)H during preparation includes mixing polyvinylpyrrolidone, sodium hydrogen carbonate, tocopherol, ascorbic acid (refer to Patent Document 1), or astaxanthin (refer to Patent Document 2).

A method known as a method of improving storage stability of ascorbic acid includes coating the surfaces of ascorbic acid powder with oil and fat such as palm oil or an, emulsifier such as a glycerin fatty acid ester (refer to Patent Document 3).

However, these methods have the problems of expensive raw materials for industrial use, the complicated operations, and the influence on product quality.

Therefore, there is demand for a method capable of simply improving storage stability of the substances at low cost.
Patent Document 1: US Patent No. 5332727
Patent Document 2: International Publication No. 2004/050099 pamphlet
Patent Document 3: Japanese Unexamined Patent Application Publication No. 54-109962

### Disclosure of Invention

### Problem to be Solved by the Invention

The present invention provides a method of improving storage stability of NAD(P)H or a salt thereof, or ascorbic acid, an ascorbic acid derivative, or a salt thereof, a composition containing such a substance, a method of producing the composition, and a method of storing the substance.

### Means for Solving the Problems

The present invention relates to the following items (1) to (6).
(1) A method of improving storage stability of NAD(P)H or a salt thereof, or ascorbic acid, an ascorbic acid derivative, or a salt thereof, which comprises allowing NAD(P)H or a salt thereof, or ascorbic acid, an ascorbic acid derivative, or a salt thereof to coexist with an L-arginine acidic amino acid salt.
(2) A method of producing a composition containing NAD(P)H or a salt thereof, or ascorbic acid, an ascorbic acid derivative, or a salt thereof, which comprises a step of mixing NAD(P)H or a salt thereof, or ascorbic acid, an ascorbic acid derivative, or a salt thereof with an L-arginine acidic amino acid salt.
(3) The method according to Item(2) in which the composition is a preparation.
(4) A composition comprising NAD(P)H or a salt thereof, or ascorbic acid, an ascorbic acid derivative, or a salt thereof and an L-arginine acidic amino acid salt.
(5) A preparation comprising NAD(P)H or a salt thereof, or ascorbic acid, an ascorbic acid derivative, or a salt thereof and an L-arginine acidic amino acid salt.
(6) A method of storing NAD(P)H or a salt thereof, or ascorbic acid, an ascorbic acid derivative, or a salt thereof, which comprises storing NAD(P)H or a salt thereof, or ascorbic acid, an ascorbic acid derivative, or a salt thereof in the presence of an L-arginine acidic amino acid salt.

### Effect of the Invention

The present invention can provide a method of improving storage stability of NAD(P)H or a salt thereof, or ascorbic acid, an ascorbic acid derivative, or a salt thereof. The present invention can also provide a composition comprising such a substance with improved storage stability, a process for producing the composition, or a method of storing the substance.

### Best Mode for Carrying Out the Invention

NAD(P)H or a salt thereof, or ascorbic acid, an ascorbic acid derivative, or a salt thereof, and an L-arginine acidic amino acid salt used in the present invention may be obtained from nature products, obtained using microorganisms or a treated product thereof, or obtained by a chemical synthesis method. The substance may be a purified product or a crude product. Further, a commercially available product may be used.

A salt of NAD(P)H may be any salt as long as it is pharmaceutically allowable. Examples of the salt include alkali metal salts such as a sodium salt and a potassium salt; inorganic acid salts such as a hydrochloride, a sulfate, and a phosphate; and organic acid salts such as an acetate, a,maleate, a fumarate, a citrate, a lactate, and a methanesulfonate.

As an ascorbic acid derivative used in the present invention, an L-ascorbic acid fatty acid ester, an L-ascorbic acid phosphate ester, or an L-ascorbic acid sulfate ester can be used. Examples of a fatty acid of the L-ascorbic acid fatty acid ester include caproic acid, heptylic acid, caprylic acid, capric acid, lauric acid, myristic acid, palmitic acid, stearic acid, and arachidic acid.

As a salt of ascorbic acid or an ascorbic acid derivative, a metal salt such as a sodium salt, a potassium salt, a calcium salt, or a magnesium salt can be used.

As an acid amino acid salt of an L-arginine acidic amino acid salt used in the present invention, a glutamate or an aspartate can be used.

The water content of the L-arginine acidic amino acid salt is preferably as low as possible, but is preferably 3% by weigh or less, more preferably 1% by weight or less, and even more preferably 0.3% by weight or less.

The storage stability of NAD(P)H or a salt thereof, or ascorbic acid, an ascorbic acid derivative, or a salt thereof can be improved by allowing NAD(P)H or a salt thereof, or ascorbic acid, an ascorbic acid derivative, or a salt thereof to coexist with the L-arginine acidic amino acid salt.

NAD(P)H or a salt thereof, or ascorbic acid, an ascorbic acid derivative, or a salt thereof may be allowed to coexist with the L-arginine acidic amino acid salt by any method. For example, a method of mixing NAD(P)H or a salt thereof, or ascorbic acid, an ascorbic acid derivative, or a salt thereof with the L-arginine acidic amino acid salt is preferably used.

A substance which is generally used in the field of pharmaceutical, food, or feed and which does not adversely affect an improvement in storage stability of NAD(P)H or a salt thereof, or ascorbic acid, an ascorbic acid derivative, or a salt thereof may be further allowed to coexist.

When these substances are allowed to coexist, the substances are preferably allowed to coexist without being dissolved in a solvent such as an aqueous solvent such as water, an aqueous inorganic salt solution, or a buffer solution, an alcohol such as methanol, ethanol, or glycerol, or a mixture thereof.

When the substances are allowed to coexist by mixing, the water content of the resultant mixture preferably does not exceed 5% by weight and more preferably does not exceed 3% by weight.

The amount of the L-arginine acidic amino acid salt is preferably 0.1 part by weight or more, more preferably 1 part by weigh or more, even more preferably 5 parts by weight or more, and most preferably 15 parts by weight or more, relative to 1 part by weight of NAD(P)H or a salt thereof, or ascorbic acid, an ascorbic acid derivative, or a salt thereof.

The storage stability of NAD(P)H or a salt thereof, or ascorbic acid, an ascorbic acid derivative, or a salt thereof can be known as the residual ratio of the substance.

With respect to the residual ratio of NAD(P)H or a salt thereof, the amount of NAD(P)H or a salt thereof is quantitatively determined by, for example, a usual method such as a method of measuring absorbance with a spectrophotometer before and after storage for a predetermined period, and the residual ratio of NAD(P)H or a salt thereof can be determined as a percentage of the amount of NAD(P)H or a salt thereof after the storage relative to the amount of NAD(P)H or a salt thereof before the storage.

With respect to the residual ratio of ascorbic acid, an ascorbic acid derivative, or a salt thereof, the amount of ascorbic acid, an ascorbic acid derivative, or a salt thereof is quantitatively determined by, for example, a usual method such as an indophenol titration method before and after storage for a predetermined period, and the residual ratio of ascorbic acid, an ascorbic acid derivative, or a salt thereof can be determined as a percentage of the amount of ascorbic acid, an ascorbic acid derivative, or a salt thereof after the storage relative to the amount of ascorbic acid, an ascorbic acid derivative, or a salt thereof before the storage.

The composition of the present invention may be a composition containing NAD(P)H or a salt thereof, or ascorbic acid, an ascorbic acid derivative, or a salt thereof, and the L-arginine acidic amino acid salt, but is preferably a composition with a water content of 5% by weight or less and more preferably a composition with a water content of 3% by weight or less.

The composition of the present invention can be prepared by mixing NAD(P)H or a salt thereof, or ascorbic acid, an ascorbic acid derivative, or a salt thereof with the L-arginine acidic amino acid salt, and if required, mixing with a substance used in the field of pharmaceutical, food, or feed.

The composition of the present invention may be used as it is as a medicine, food, feed, or a raw material thereof.

The composition may be prepared as a preparation, preferably a solid preparation, together with preparation bases used in the field of pharmaceutical or food.

Examples of the preparation include a tablet, a capsule, a suppository, a pill, a powder, and a granule.

The preparation bases include an excipient, a disintegratant, a binder, and a lubricant.

Examples of the excipient include maltose, trehalose, mannitol, reduced malt sugar syrup, lactitol, xylitol, sorbitol, erythritol, crystalline cellulose, and low-substitution-degree hydroxypropyl cellulose.

Examples of the disintegratant include carboxymethyl cellulose, carboxymethyl cellulose calcium, carboxymethyl cellulose sodium, crospovidone, sodium croscarmellose, sodium glycolate, and starch such as corn starch, potato starch, partially pregelatinized starch.

Examples of the binder include polyvinylpyrrolidone, pullulan, methyl cellulose, hydroxypropyl cellulose, polyvinyl alcohol, gelatin, and agar.

Examples of the lubricant include sucrose fatty acid esters, stearic acid, metal stearates such as magnesium stearate, calcium stearate, and sodium stearyl fumarate, glycerol fatty acid esters, and hardened oil and fat.

The ratio of the above-mentioned excipient, the disintegratant, the binder, or the lubricant in the composition of the present invention is not particularly limited as long as it is in the range of amounts generally used for preparations.

Besides the preparation bases, the composition may comprise a sweetener, an acidulant, a coloring agent, a flavor, an antioxidant, and a plasticizer, if required.

Examples of the sweetener include saccharin sodium, dipotassium glycyrrhizinate, aspartame, stevia, thaumatin, sucralose, glucose, fructose, and saccharose.

Examples of the acidulant include citric acid, tartaric acid, and malic acid.

Examples of the coloring agent include Food Yellow No. 5, Food Red No. 2, and Food Blue No. 2.

Examples of the flavor include lemon flavor, lemon lime flavor, grapefruit flavor, apple flavor, and orange flavor.

Examples of the antioxidant include tocopherol and cysteine hydrochloride.

Examples of the plasticizer include calcium phosphate, calcium hydrogen phosphate, and fine silicon dioxide powder.

The ratio of the above-mentioned sweetener, the acidulant, the coloring agent, the flavor, the antioxidant, or the plasticizer in the composition of the present invention is not particularly limited as long as it is in the range of amounts generally used for formulations.

Besides the above-described ingredients, a carbohydrate such as dextrin, niacin, vitamins [excluding vitamin C (ascorbic acid) when the substrate in coexistence with the L-arginine acidic amino acid salt is ascorbic acid or an ascorbic acid derivative], minerals such as sodium, a desiccant such as fine silicon dioxide powder, and an anticaking agent such as calcium silicate, synthetic aluminum silicate, or talc may be added.

The content of NAD(P)H or a salt thereof, or ascorbic acid, an ascorbic acid derivative, or a salt thereof in the composition of the present invention is preferably 0.01 to 90% by weight, more preferably 0.1 to 70% by weight, even more preferably 0.1 to 50% by weight, and most preferably 1 to 30% by weight.

The content of the L-arginine acidic amino acid salt in the composition of the present invention is preferably 10 to 99.99% by weight, more preferably 30 to 99.9% by weight, even more preferably 50 to 99.9% by weight, and most preferably 70 to 99% by weight.

In order to effectively improve the storage stability of NAD(P)H or a salt thereof, or ascorbic acid, an ascorbic acid derivative, or a salt thereof, the content of the L-arginine acidic amino acid salt is preferably 0.1 part by weight or more, more preferably 1 part by weight or more, even more preferably 5 parts by weight or more, and most preferably 15 parts by weight or more, relative to 1 part by weight of the substance.

The production method will be described with reference to a tablet and a hard capsule as an example of the composition of the present invention.

For example, a tablet can be produced by a method (hereinafter, referred to as a "direct tableting method") in which NAD(P)H or a salt thereof, or ascorbic acid, an ascorbic acid derivative, or a salt thereof, the L-arginine acidic amino acid salt, the preparation bases, and, if required, the above-described ingredients other than the preparation bases are mixed, and the resultant mixture is compression-molded; a method in which NAD(P)H or a salt thereof, or ascorbic acid, an ascorbic acid derivative, or a salt thereof, the L-arginine acidic amino acid salt, the preparation bases, and, if required, some of the above-described ingredients other than the preparation bases are granulated, the resultant granules are mixed with the remaining ingredients, and the resultant mixture is compression-molded; or a method in which NAD(P)H or a salt thereof, or ascorbic acid, an ascorbic acid derivative, or a salt thereof, the L-arginine acidic amino acid salt, the preparation bases, and, if required, all of the above-described ingredients other than the preparation bases are granulated, and the resultant granules are compression-molded. However, the direct tableting method is preferably used.

The apparatus used for compression molding is not particularly limited, and for example, a rotary compression molding machine, or a compressor such as a hydraulic press machine can be used.

When a tablet is produced as an enteric coated tablet, a tablet can be produced by coating the surface of a tablet prepared by compression molding with a base generally used for enteric coating, such as a shellac solution, a zein solution, or cellulose acetate, using a coating pan. When a tablet is produced as a sublingual tablet, the tablet can be produced by tableting under a low pressure.

When a hard capsule is produced, a hard capsule can be produced by a method in which NAD(P)H or a salt thereof, or ascorbic acid, an ascorbic acid derivative, or a salt thereof, the L-arginine acidic amino acid salt, the preparation bases, and, if required, the above-described ingredients other than the preparation bases are mixed, and the resultant mixture is encapsulated in a capsule by a encapsulator. The hard capsule is sealed by a general method, if required.

When the hard capsule is used as an enteric hard capsule, the enteric hard capsule can be produced by coating the surface of the hard capsule prepared by the above-mentioned method with a base generally used for coating in producing enteric coated tablets, such as a shellac solution, a zein solution, or cellulose acetate, using an ordinary method.

The composition of the present invention may be stored by any method which can store NAD(P)H or a salt thereof, or ascorbic acid, an ascorbic acid derivative, or a salt thereof. However, the composition is preferably stored by standing in a light-shielded container and more preferably stored in a container which can be hermetically sealed.

The storage temperature may be any temperature within an ordinary range, but is preferably 50°C or less.

When the composition of the present invention is administered to a human or nonhuman animal, the dosage depends on the purpose of administration, the administration form, and the age, weight, and symptoms of a human or nonhuman animal to be administered.

For example, when the composition of the present invention is a composition comprising NAD(P)H or a salt thereof, NAD(P)H or a salt thereof is orally administered one to three times a day in a dosage of preferably 1 to 100 mg and more preferably 3 to 30 mg per adult.

When the composition of the present invention is a composition comprising ascorbic acid, an ascorbic acid derivative, or a salt thereof, or ascorbic acid, an ascorbic acid derivative, or a salt thereof is administered several times a day in a dosage of preferably 10 to 1500 mg and more preferably 30 to 1000 mg per adult.

### EXAMPLE 1

L-arginine L-glutamate (manufactured by Kyowa Hakko Kogyo Co., Ltd, the same shall apply hereinafter) and D-mannitol [D-mannitol (for oral administration), manufactured by Nikken Chemical and Synthetic Industry Co., Ltd., the same shall apply hereinafter] were dried at 70°C for 60 minutes using a constant-temperature dryer. The dried L-arginine L-glutamate and D-mannitol were dried at 105°C for 240 minutes using a constant-temperature dryer, and the water content was calculated from a weight difference before and after drying. As a result, the water contents of the L-arginine L-glutamate and D-mannitol obtained by drying at 70°C were 0.3% by weight and 0.1% by weight, respectively.

Then, 96.6 g of the L-arginine L-glutamate with the water content of 0.3% by weight was mixed and stirred with 3.4 g of NADH (manufactured by Kyowa Hakko Kogyo Co., Ltd., the same shall apply hereinafter) to prepare mixed powder A.

The mixed powder A was dried at 105°C for 240 minutes using a constant-temperature dryer, and the water content was calculated from a weight difference before and after drying. As a result, the water content was 0.3% by weight.

Then, 96.6 g of the D-mannitol with the water content of 0.1% by weight was mixed and stirred with 3.4 g of NADH to prepare mixed powder B.

As a result of determination of the water content of the mixed powder B by the same method as for the mixed powder A, the water content was 0.1% by weight.

Also, the NADH content of each of the mixed powders A and B was determined by high-performance liquid chromatography (HPLC) under the following conditions:
HPLC conditions
Column: LiChroCART 250-4 LiChrospher 100RP-18e 5 µm, 4 mm in diameter × 25 cm (manufactured by Merck & Co., Ltd.)
Guard column: LiChroCART 4-4 LiChrospher 100RP-18e 5 µm (manufactured by Merck & Co., Ltd.)
Mobile phase: prepared by dissolving 13.58 g of tetra-n-butylammonium hydrogen sulfate and 68.04 g of potassium dihydrogen phosphate in water, adjusting the pH of the resultant solution to 6.0 with potassium hydroxide, controlling the total amount to 4715 ml with water, adding 285 ml of acetonitrile, and then stirring and degassing the resultant mixture.
Sample: prepared by dissolving 200 mg of the mixed powder in a 0.01 mol/l sodium carbonate solution and allowing the amount to be 100 ml.
Detection wavelength: 254 nm
Column temperature: 25°C
Mobile phase flow rate: 1.0 ml/min

Each of the mixed powders A and B was placed in a petri dish half covered and allowed to stand at 25°C and a relative humidity of 43% for 5 hours in an incubator. After standing, each of the powders was packed in an aluminum-deposited bag, and the bag was sealed with a sealer, followed by storage at 60°C for 14 days. The NADH content in each mixed powder was determined after the storage by the same method as that before the storage.

The residual ratio of NADH after the storage was calculated from the NADH contents of each mixed powder before and after the storage.

As a result, the residual ratio of NADH in the mixed powder A containing the L-arginine L-glutamate was 83%, while the residual ratio of NADH in the mixed powder B containing the D-mannitol was 66%.

Therefore, the storage stability of NADH was improved by allowing NADH to coexist with L-arginine L-glutamate.

### EXAMPLE 2

First, 2400 g of the L-arginine L-glutamate with the water content of 0.3% by weight prepared in Example 1, 102 g of NADH, 450 g of crystalline cellulose (Avicel FD101 manufactured by Asahi Kasei Corporation, the same shall apply hereinafter), 30 g of magnesium stearate (manufactured by San Ei Gen F.F.I., Inc., the same shall apply hereinafter), and 18 g of calcium phosphate (manufactured by Taihei Chemical Industrial Co., Ltd.) were mixed and stirred to prepare mixed powder C.

Mixed powder D was prepared by the same method as that for producing the mixed powder C except that 2400 g of the D-mannitol with the water content of 0.1% by weight prepared in Example 1 was used.

As a result of determination of the water contents of the mixed powders C and D according to the method described in Example 1, the water contents of the mixed powders C and D were 1.3% by weight and 0.5% by weight, respectively.

Each of the mixed powders C and D was tableted with rotary tablet press VIRGO524 (manufactured by Kikusui Seisakusho Ltd., the same shall apply hereinafter) to prepare tablets C and D of 9 mm in diameter and about 300 mg/tablet.

As a result of determination of the water contents of the tablets C and D according to the method described in Example 1, the water contents of the tablets C and D were 1.3% by weight and 0.5% by weight, respectively.

The tablets C and D were pulverized, and the NADH contents in the tablets were measured using the resultant powders according to the method described in Example 1.

The tablets C and D were placed in respective containers, sealed, and then stored at 60°C for 14 days.

The NADH contents of the tablets were measured after the storage by the same method as that before the storage.

The residual ratio of NADH was calculated from the NADH contents in the tablets before and after the storage. As a result, the residual ratio of NADH in the tablets C containing L-arginine L-glutamate was 84%, while the residual ratio of NADH in the tablets D containing D-mannitol was 69%.

Therefore, the storage stability of NADH was improved by allowing NADH in tablets to coexist with L-arginine L-glutamate.

### EXAMPLE 3

First, 3960 g of the L-arginine L-glutamate with the water content of 0.3% by weight prepared in Example 1, 200 g of NADH, and 40 g of silicon dioxide were mixed and stirred to prepare a mixture, and the resultant mixture was charged in a encapsulator and encapsulated in 20,000 of No. 2 hard capsules made of gelatin to prepare hard capsules. The surfaces of the resultant hard capsules were coated with a zein solution using high coater HCT-48 model (Freund Industry Co., Ltd., the same shall apply hereinafter) to produce 20000 enteric capsules containing 10 mg of NADH.

### EXAMPLE 4

The surfaces of the tablets C prepared in Example 2 were coated with a shellac solution using high coater HCT-48 model to produce enteric tablets.

### EXAMPLE 5

First, 2400 g of the L-arginine L-glutamate with the water content of 0.3% by weight prepared in Example 1, 102 g of L-ascorbic acid (manufactured by Japan Co., Ltd., DSM Nutrition), 450 g of crystalline cellulose, 30 g of magnesium stearate, and 18 g of calcium phosphate were mixed and stirred to prepare mixed powder E.

Mixed powder F was prepared by the same method as that for producing the mixed powder E except that 2400 g of the D-mannitol with the water content of 0.1% by weight prepared in Example 1 was used.

As a result of determination of the water contents of the mixed powders E and F according to the method described in Example 1, the water contents of the mixed powders E and F were 2.0% by weight and 1.1% by weight, respectively.

Each of the mixed powders E and F was tableted with rotary tablet press VIRGO524 to prepare tablets E and F of 9 mm in diameter and about 300 mg/tablet.

As a result of determination of the water contents of the tablets E and F according to the method described in Example 1, the water contents of the tablets E and F were 2.1% by weight and 1.1% by weight, respectively.

Each of the tablets E and F was pulverized and added to a metaphosphoric acid-acetic acid solution [prepared by dissolving 15 g of metaphosphoric acid (Kishida Chemical Co., Ltd.) and 40 ml of glacial acetic acid (Kishida Chemical Co., Ltd.) in distilled water so that the amount was 500 ml] so that the total amount was 100 ml to prepare a sample solution.

Then, 40 mg of 2,6-dichloroindophenol sodium n-hydrate (Kishida Chemical Co., Ltd.) was dissolved in distilled water so that the total amount was 100 ml to prepare a 2,6-dichloroindophenol sodium solution.

The sample solution was added dropwise to the 2,6-dichloroindophenol sodium solution to determine the amount of the sample solution required until the purple color of the solution became transparent.

A calibration curve was formed by the same operation as described above except that an L(+)-ascorbic acid reagent (Kishida Chemical Co., Ltd.) was used, and the amount of ascorbic acid in the sample solution was calculated from the amount of the sample solution required until the purple of the solution became transparent using the calibration curve.

The tablets E and F were placed in containers, sealed, and then stored at 60°C for 30 days.

The L-ascorbic acid contents in the tablets were measured after the storage by the same method as that before the storage.

The residual ratio of L-ascorbic acid was calculated from the L-ascorbic acid contents before and after the storage. As a result, the residual ratio in the tablets E containing L-arginine L-glutamate was 98%, while the residual ratio in the tablets F containing D-mannitol was 91%.

Therefore, the storage stability of L-ascorbic acid was improved by allowing L-ascorbic acid in tablets to coexist with L-arginine L-glutamate.

### Industrial Applicability

The present invention can provide a method of improving storage stability of NAD(P)H or a salt thereof, or ascorbic acid, an ascorbic acid derivative, or a salt thereof. The present invention can also provide a composition containing such a substance with improved storage stability, a process for producing the composition, and a method of storing the substance.

## Claims

1. A method of improving storage stability of reduced nicotinamide adenine dinucleotide or reduced nicotinamide adenine dinucleotide phosphate [hereinafter referred to as "NAD(P)H"], or a salt thereof, or ascorbic acid, an ascorbic acid derivative, or a salt thereof, which comprises allowing NAD(P)H or a salt thereof, or ascorbic acid, an ascorbic acid derivative, or a salt thereof to coexist with an L-arginine acidic amino acid salt.

2. A process for producing a composition containing NAD(P)H or a salt thereof, or ascorbic acid, an ascorbic acid derivative, or a salt thereof, which comprises a step of mixing NAD(P)H or a salt thereof, or ascorbic acid, an ascorbic acid derivative, or a salt thereof with an L-arginine acidic amino acid salt.

3. The method according to claim 2, wherein the composition is a preparation.

4. A composition comprising NAD(P)H or a salt thereof, or ascorbic acid, an ascorbic acid derivative, or a salt thereof and an L-arginine acidic amino acid salt.

5. A preparation comprising NAD(P)H or a salt thereof, or ascorbic acid, an ascorbic acid derivative, or a salt thereof and an L-arginine acidic amino acid salt.

6. A method of storing NAD(P)H or a salt thereof, or ascorbic acid, an ascorbic acid derivative, or a salt thereof, which comprises storing NAD(P)H or a salt thereof, or ascorbic acid, an ascorbic acid derivative, or a salt thereof in the presence of an L-arginine acidic amino acid salt.
